# EUROPÄISCHE PATENTSCHRIFT

(11) **EP 1 002 507 B1**
(45) Veröffentlichungstag und Bekanntmachung des Hinweises auf die Patenterteilung: **19.02.2003**
(21) Anmeldenummer: 99121233.3
(22) Anmeldetag: 23.10.1999
(51) Int. Cl.: A61F 5/44, A61B 5/20

(54) **Urinmessgerät**
Device for measuring urine
Appareil de mesure d'urine

(30) Priorität: 17.11.1998 DE 29820526 U
(43) Veröffentlichungstag der Anmeldung: 24.05.2000
(73) Patentinhaber: B. Braun Melsungen AG, 34212 Melsungen (DE)
(72) Erfinder: Voges, Karl-Friedrich, 34212 Melsungen (DE); Sippel, Martin, Dr., 34212 Melsungen (DE); Collin, Rémi, 28230 Epernon (FR)
(74) Vertreter: Selting, Günther, Dipl.-Ing.

(56) Entgegenhaltungen:
- US-A- 3 928 875
- US-A- 4 334 537
- US-A- 4 490 144
- US-A- 4 846 816

## Beschreibung

Die Erfindung betrifft ein Urinmeßgerät und insbesondere ein Urinmeßgerät, welches eine Tropfkammer mit einem Antirefluxventil aufweist.

Urinmeßgeräte dienen der Aufnahme des Urins, der durch einen Katheter aus einem Patientenkörper herausläuft. In den Meßbehälter eines Urinmeßgerätes können Keime aus der Umgebung einwandern, die sich dort vermehren. Diese Keime können durch den zum Patienten führenden Verbindungsschlauch in den Patientenkörper gelangen und eine Infektion der Harnwege verursachen. Zur Vermeidung solcher Infektionen sind Urinmeßgeräte in der Regel an der Einmündung des Verbindungsschlauchs in den Meßbehälter mit einer Pasteur'schen Kammer ausgerüstet. Diese besteht aus einer Tropfkammer mit einem Tropfer auf der Zulaufseite und einem Antirefluxventil auf der Abflußseite. In der Tropfkammer wird die aus dem Verbindungsschlauch kommende Flüssigkeitssäule unterbrochen. Der Urin tropft von einer Tropferspitze ab und fällt dabei frei durch die Luft, ohne die Wände der Tropfkammer zu benetzen. Auf den trockenen Wänden können sich die Krankheitserreger nicht weiter in Richtung auf den Patienten bewegen.

Während einer Sammelphase (meist eine Stunde) hängt das Urinmeßgerät senkrecht an dem Bett des Patienten. Am Ende der Sammelphase wird die Urinmenge protokolliert und der Meßbehälter wird in den Urinbeutel entleert. Hierzu wird der Meßbehälter um mehr als 90° gekippt. Der zurückfließende Urin könnte dabei auch den Tropfer und die Innenwände der Tropfkammer benetzen, so daß die Keimschranke aufgehoben wäre. Um dies zu verhindern, ist es bekannt, am Boden der Tropfkammer ein Antirefluxventil vorzusehen.

Ein Antirefluxventil in Verbindung mit einer Tropfkammer ist bekannt aus DE-PS 25 43 778. Hierbei besteht das Antirefluxventil aus einer eine seitliche Öffnung der Tropfkammer verschließenden Ventilklappe, die von einem Halteglied in die Schließstellung vorgespannt ist. Das Halteglied und die Ventilklappe bilden ein Rückschlagventil. Dieses Rückschlagventil besteht aus mindestens zwei Teilen, von denen das Halteglied eine komplexe Form hat. Daher ist die Herstellung relativ aufwendig.

Ein Antirefluxventil ist bekannt aus US 4 334 537. Diese Antirefluxventil enthält eine Ventilkammer mit einem Ventilsitz, gegen den ein Ventilteller gezogen wird. Der Ventilteller ist einstückig mit einer Haltestange geformt, welche an den Enden federnde Ankerelemente aufweist. Hierbei erfordert die Herstellung des komplexen Ventilkörpers einen erheblichen Aufwand. Außerdem ist zu berücksichtigen, daß mit dem Urin auch Festkörper (Blutkoagel, Grieß) transportiert werden können, die sich in dem Ventil festsetzen und dieses offenhalten.

Ein Urinmeßgerät, bei dem am unteren Ende einer Meßkammer ein Rückschlagventil vorgesehen ist, ist bekannt aus DE 41 37 074 A1. Das Rückschlagventil besteht aus einer Ventilplatte, die gegen einen an der Unterseite der Ventilöffnung vorgesehenen umlaufenden Ventilsitz drückt. Über die Befestigung der Ventilplatte und ihre Funktion werden keine Angaben gemacht.

Ein Urinmeßgerät nach dem Oberbegriff des Patentanspruchs 1 ist in US-A-4,490,144 beschrieben. Dieses Urinmeßgerät enthält eine Tropfkammer, die über einer Feinmeßkammer angeordnet ist. In die Tropfkammer mündet ein Tropfer. An der Unterseite der Tropfkammer befindet sich ein als Rückflußsperre dienendes Ventil, dessen Ventilplatte von sich anstauender Flüssigkeit gegen einen Ventilsitz hochgedrückt wird und diesen verschließt. Der Ventilsitz ist an einem separaten Bauteil vorgesehen, das mit dem unteren Rohrende der Ventilkammer verbunden ist.

US-A-4,846,816 beschreibt eine Urinableitungsvorrichtung, bei der ein Zulaufrohr mit einem Beutel über ein Ventil, das eine Rückflußsperre bildet, verbunden ist. Das Ventil enthält eine Ventilplatte, die als frei bewegliches Losteil ausgebildet ist und in dem Ventilgehäuse geführt ist. Das Ventilgehäuse besteht auch hier aus zwei ineinander gesteckten Rohrteilen.

Der Erfindung liegt die Aufgabe zugrunde, ein Urinmeßgerät mit Tropfkammer und Antirefluxventil zu schaffen, bei dem die Gefahr von Fehlfunktionen durch festgehaltene Feststoffkörper ausgeschlossen ist und welches in Herstellung und Montage einfach und kostengünstig ist.

Die Lösung dieser Aufgabe erfolgt erfindungsgemäß mit den im Patentanspruch 1 angegebenen Merkmalen.

Die Erfindung geht von dem Gedanken aus, daß das Ventil normalerweise offen sein muß, andererseits in Schließrichtung aber dicht sein soll. Ferner soll das Ventil zur Verhinderung des Festsetzens von Blutkoagel oder Grieß möglichst groß dimensioniert sein. Erfindungsgemäß weist das Urinmeßgerät eine Ventilplatte auf, die aus einem frei beweglichen Losteil besteht. Dies bedeutet, daß die Ventilplatte an keiner Stelle aufgehängt oder an anderen Konstruktionsteilen fest angebracht ist. Es handelt es sich um ein lose bewegliches Teil. Die Ventilplatte ist eine flache Platte, die keine aus der Plattenebene abstehenden Ansätze o.dgl. aufweist. Die Ventilplatte legt sich von unten her gegen einen Ventilsitz, falls eine äußere Krafteinwirkung durch zurückdrückende Flüssigkeit auftritt. Im unbelasteten Zustand befindet sich die Ventilplatte von dem Ventilsitz entfernt, so daß die Ventilöffnung offen ist. Dies bedeutet, daß der herabtropfende Urin die Ventilöffnung praktisch drucklos durchfließen kann. Wird das Urinmeßgerät zum Zwecke des Entleerens der Meßkammer oder aus anderen Gründen schräggestellt, dann drückt der im Meßbehälter enthaltene Urin die Ventilplatte gegen den Ventilsitz, wodurch der Ventilsitz abgedichtet wird. Dadurch wird verhindert, daß rückfließender Urin in die Tropfkammer gelangt und deren Wände benetzt. Da die Wände der Tropfkammer trocken bleiben, bilden sie keine zum Patienten führende Brücken für eine Keimwanderung.

Das Ventil, mit Ausnahme der Ventilplatte, ist integraler Bestandteil eines Meßgeräte-Gehäuses, das die Tropfkammer enthält. Hierbei sind bei der Montage keine zusätzlichen Bestandteile in das Gehäuse einzubringen. Die Ventilplatte wird lediglich in den hierfür bestimmten Hohlraum eingelegt, ohne dass eine Fixierung erforderlich wäre. Das Meßgeräte-Gehäuse weist zwei zusammengesetzte Gehäuseteile auf. Die Ventilöffnung und die Führungselemente sind Bestandteil des einen Gehäuseteils und ein die Ventilplatte abstützender Kragarm ist Bestand des anderen Gehäuseteils. Für die Montage werden lediglich die beiden Gehäuseteile unter Zwischenfügung der Ventilplatte zusammengefügt.

Ein wesentlicher Vorteil der Erfindung besteht darin, daß das Ventil in das Meßgeräte-Gehäuse integriert ist und als einziges spezifisches Zusatzteil lediglich die Ventilplatte erfordert. Die Teile des Ventilgehäuses können einstückig in das Meßgehäuse integriert sein, so daß eine gesonderte Herstellung oder Montage entfällt. Die Ventilplatte ist eine flache Platte, die keiner besonderen dreidimensionalen Formgebung bedarf. Vielmehr kann sie aus einem geeigneten flachen Folienmaterial ausgestanzt sein. Sie besteht vorzugsweise aus Polyesterfolie. Die Ventilplatte ist relativ steif und formstabil. Sie besteht vorzugsweise aus halbstarrem Kunststoff.

Die Ventilplatte wird zwischen seitlichen Führungselementen lose gehalten. Dies bedeutet, daß die Ventilplatte an keiner Stelle eine feste Verbindung mit dem Ventilgehäuse hat. Die Führungselemente bilden gewissermaßen einen Käfig, in dem die Ventilplatte bewegbar ist. Sie sind so angeordnet, daß in jeder möglichen Stellung die Projektion der Ventilplatte die Ventilöffnung vollständig bedeckt.

Die seitlichen Führungselemente bestehen vorzugsweise aus Rippen, die von der Wand des Ventilgehäuses nach innen abstehen, wobei das Ventilgehäuse einen größeren Querschnitt hat als die Ventilöffnung. Damit steht ein großer Durchlaßquerschnitt zur Verfügung. Infolge des Fehlens elastischer Spannmittel und infolge eines großen freien Durchlaßquerschnitts besteht nicht die Gefahr, daß im Urin enthaltene Feststoffe die Funktion des Ventils behindern.

Die Querschnittsfläche der Ventilöffnung ist vorzugsweise um mindestens 70 % größer als der Innenquerschnitt des Tropfers. Auf diese Weise ist eine hinreichende Größe der Ventilöffnung zur Verhinderung des Zusetzens sichergestellt.

Im folgenden wird unter Bezugnahme auf die Zeichnungen ein Ausführungsbeispiel der Erfindung näher erläutert.

Es zeigen:

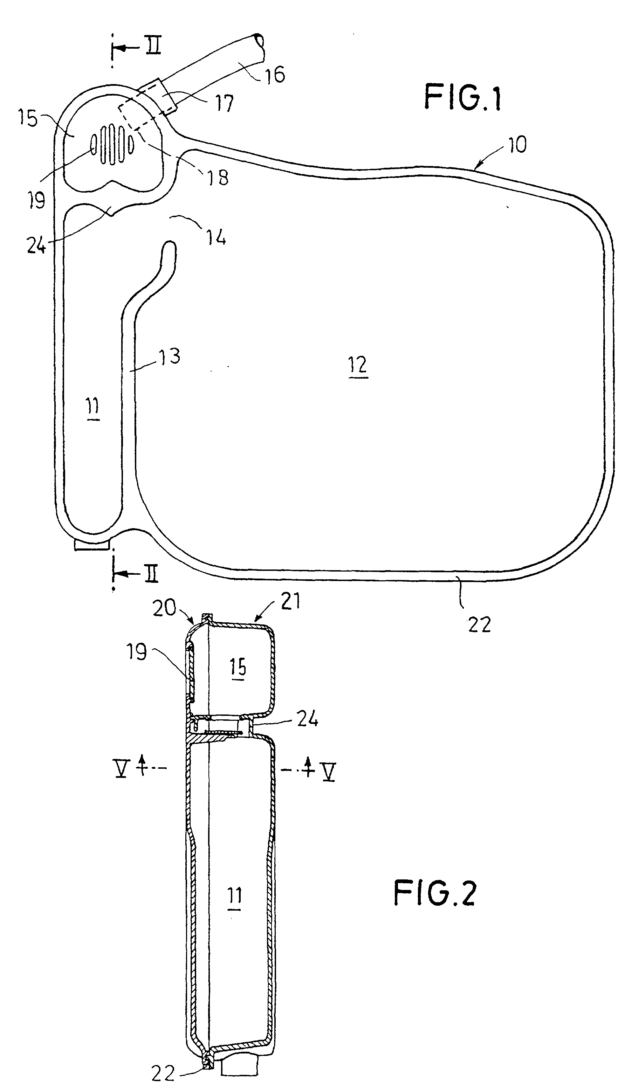

- Fig. 1: eine Vorderansicht des Urinmeßgerätes,
- Fig. 2: einen Schnitt entlang der Linie II-II von Fig. 1,
- Fig. 3: eine vergrößerte Darstellung des Ventilbereichs aus Fig. 2, wobei in der Sammelphase das Ventil geöffnet ist,
- Fig. 4: in gleicher Darstellung wie Fig. 2 die Entleerungsphase, in der das Ventil geschlossen ist, und
- Fig. 5: einen Schnitt entlang der Linie V-V von Fig. 2.

Das Urinmeßgerät weist einen Meßbehälter 10 auf, der aus einer Feinmeßkammer 11 und einer Grobmeßkammer 12 besteht. Die Feinmeßkammer und die Grobmeßkammer sind durch einen Steg 13 getrennt und durch einen Überlauf 14 am oberen Ende des Steges 13 miteinander verbunden.

Über der Feinmeßkammer 11 ist eine Tropfkammer 15 angeordnet, in die ein vom Patienten kommender Zulaufschlauch 16 mündet. Der Zulaufschlauch 16 ist in einer Schlauchfixierung 17 befestigt. Der Zulaufschlauch ist so eingeklebt, daß sein vorderes Ende einige Millimeter in die Tropfkammer 15 hineinragt und einen Tropfer 18 bildet. An der Vorderseite der Tropfkammer 15 ist ein Belüftungsfilter 19 angebracht, der mit der Umgebungsluft in Verbindung steht.

Die Tropfkammer 15, die Feinmeßkammer 11 und die Grobmeßkammer 12 sind Bestandteil eines aus zwei Gehäuseteilen bestehenden Meßgeräte-Gehäuses. Der eine Gehäusebestandteil 20 bildet das Vorderteil und der andere Gehäusebestandteil 21 bildet das Rückenteil. Vorderteil und Rückenteil sind durch eine umlaufende Nut-Feder-Verbindung 22 unter Klebung bzw. Schweißung verbunden. Die Nut-Feder-Verbindung 22 erstreckt sich umlaufend um das gesamte Meßgeräte-Gehäuse sowie auch entlang der Innenwände.

In dem Übergangsbereich zwischen Tropfkammer 15 und Feinmeßkammer 11 ist das Ventilgehäuse 24 ausgebildet. Dieses besteht aus zusammengefügten Komponenten der beiden Gehäuseteile 20,21, die jeweils einstückige Kunststoff-Formteile bilden.

Das Ventilgehäuse 24 enthält eine horizontale Zwischenwand 25, welche die Bodenwand der Tropfkammer 15 bildet. In der Zwischenwand 25 ist die kreisrunde Ventilöffnung 26 enthalten. Der untere Rand der Ventilöffnung 26 bildet den wulstartigen umlaufenden Ventilsitz 27.

Mit Abstand unterhalb der Ventilöffnung 26 ist ein Kragarm 28 angeordnet, der von der zum Gehäuseteil 20 gehörenden Vorderwand 29 nach innen absteht und etwa parallel zur Zwischenwand 25 verläuft. Dieser Kragarm bildet eine Tragfläche oder Auffangfläche für die Ventilplatte 30.

Die Ventilplatte 30 besteht aus einer Kunststoffolie, vorzugsweise Polyesterfolie. Ihr spezifisches Gewicht ist größer als dasjenige von Urin. Es handelt sich um eine flache ausgestanzte runde Platte, die lose in das Ventilgehäuse 24 eingelegt ist. Die Ventilplatte 30 wird durch seitliche Führungselemente 31 in Form von Rippen, die von der Wand des Ventilgehäuses 24 nach innen abstehen, zentriert gehalten. Die Führungselemente 31 sind, ebenso wie die Zwischenwand 25, Bestandteil des rückwärtigen Gehäuseteils 21.

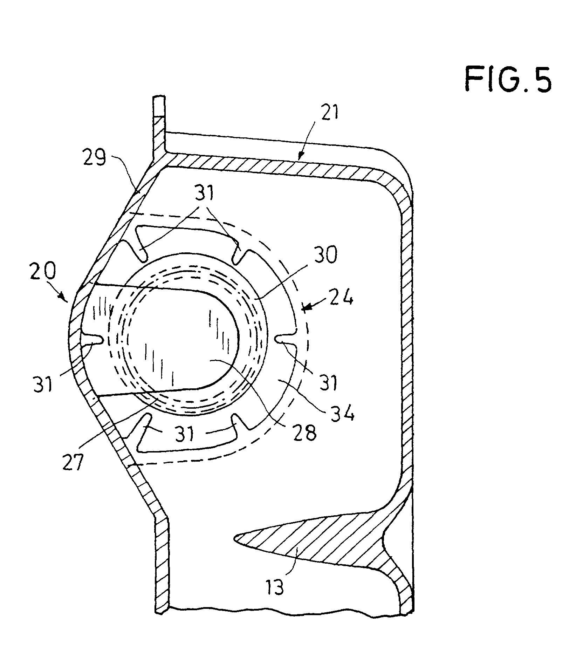

Figur 5 zeigt einen Blick von unten in das Ventilgehäuse 24 aus der Feinmeßkammer 11 heraus. Dabei sind die radial angeordneten rippenförmigen Führungselemente 31 erkennbar, von denen hier sechs vorgesehen sind. Diese Führungselemente halten die Ventilplatte 30 in Überdeckung mit der von dem Ventilsitz 27 umschlossenen Ventilöffnung. Die Ventilplatte 30 ist in Figur 5 nicht in der exakten Mittellage dargestellt, sondern seitlich versetzt. In jedem Fall überdeckt sie die Ventilöffnung vollständig. Aus Figur 5 ist ebenfalls erkennbar, daß der Kragarm 28 nur einen Teil der Fläche der Ventilplatte 30 untergreift. Auf einem Winkelbereich von mehr als 270° überragt die Ventilplatte 30 die Kontur des Kragarmes 28.

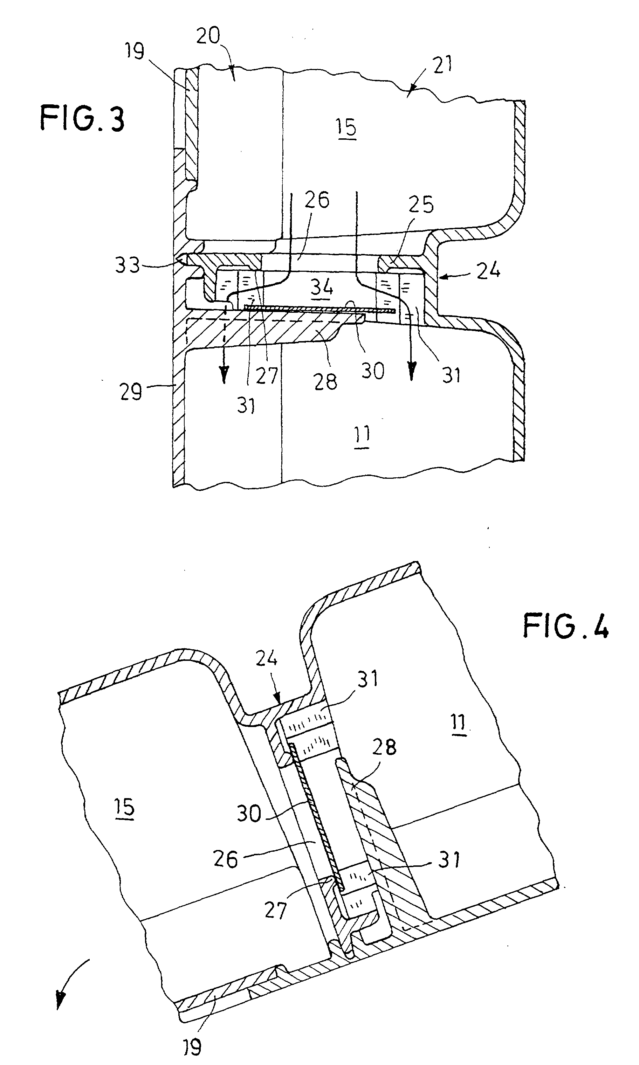

Wie aus Figur 3 hervorgeht, ragt die Zwischenwand 25, die Bestandteil des rückwärtigen Gehäuseteils 21 ist, in eine Nut 33 hinein, die an der Innenseite der Vorderwand 29 vorgesehen ist. Sämtliche Führungselemente 31 sind Bestandteil des rückwärtigen Gehäuseteils 21.

Die von dem Ventilgehäuse 24 umschlossene Ventilkammer 34, in die die Führungselemente 31 radial hineinragen, hat einen Durchmesser, der deutlicher größer ist als derjenige der Ventilplatte 30. Daher kann in dem in Figur 3 dargestellten Zustand, in dem die Ventilplatte 30 durch ihr Gewicht auf dem Kragarm 28 ruht, Flüssigkeit an der Ventilplatte 30 entlang und zwischen den Führungselementen 31 hindurch in die Feinmeßkammer 11 fließen.

Wird das Urinmeßgerät gekippt, um die Meßkammern zu entleeren, drückt die in der Feinmeßkammer 11 enthaltene Flüssigkeit die Ventilplatte 30 gegen den Ventilsitz 27, wie dies in Figur 4 dargestellt ist. Dadurch wird verhindert, daß Flüssigkeit von der Feinmeßkammer 11 in die Tropfkammer 15 zurückfließen kann.

## Patentansprüche

1. Urinmeßgerät mit einer Tropfkammer (15), in die ein Tropfer (18) mündet und die im Boden ein Ventil aufweist, wobei das Ventil aus einer Ventilöffnung (26), die an ihrer Unterseite einen umlaufenden Ventilsitz (27) aufweist, und einer Ventilplatte (30) besteht, und das Ventil mit einer Feinmeßkammer (11) verbunden ist,
**dadurch gekennzeichnet,**
**dass** die Tropfkammer (15), die Feinmeßkammer (11) und eine Grobmeßkammer (12) in einem zweiteiligen Meßgeräte-Gehäuse ausgebildet sind, welches aus einem ein Vorderteil bildenden Gehäuseteil (20) und einem ein Rückenteil bildenden Gehäuseteil (21) besteht, und dass die Ventilöffnung (26) und die Führungselemente (31) Bestandteil des einen Gehäuseteils (21) und ein die Ventilplatte (30) abstützender Kragarm (28) Bestandteil des anderen Gehäuseteils (20) sind.

2. Urinmeßgerät nach Anspruch 1, **dadurch gekennzeichnet, daß** die Ventilplatte (30) durch einen Kragarm (28) abgestützt ist.

3. Urinmeßgerät nach Anspruch 1 oder 2, **dadurch gekennzeichnet, daß** die seitlichen Führungselemente (31) aus Rippen bestehen, die von der Wand des einen Gehäuseteils (21) in ein von beiden Gehäuseteilen gebildetes Ventilgehäuse (24) nach innen abstehen, welches einen größeren Querschnitt hat als die Ventilöffnung (26).

4. Urinmeßgerät nach einem der Ansprüche 1 bis 3, **dadurch gekennzeichnet, daß** die Querschnittsfläche der Ventilöffnung (26) um mindestens 70 % größer ist als der Innenquerschnitt des Tropfers (18).

## Claims

1. Urine measuring device comprising a drip chamber (15) with a dropper (18) leading into said drip chamber and said drip chamber having a valve arranged on the bottom, said valve having a valve opening (26) with a circumferential valve seat (27) on its underside, and a valve plate (30), and the valve being connected with a fine measuring chamber (11),
**characterized in that**
the drip chamber (15), the fine measuring chamber (11) and a coarse measuring chamber (12) are formed in a bipartite measuring device housing which comprises a housing part (20) forming a front part and a housing part (21) forming a back part, and the valve opening (26) and the guide elements (31) are components of the one housing part (21), and a bracket (28) supporting the valve plate (30) is a component of the other housing part (20).

2. Urine measuring device according to claim 1, **characterized in that** the valve plate (30) is supported by a bracket (28).

3. Urine measuring device according to claim 1 or 2, **characterized in that** the lateral guide elements (31) comprise ribs projecting inwardly from the wall of the one housing part (21) into a valve housing (24) formed by the two housing parts, which valve housing (24) has a larger cross-section than the valve opening (26).

4. Urine measuring device according to one of claims 1 to 3, **characterized in that** the cross-sectional area of the valve opening (26) is at least 70 % larger than the inner cross-section of the dropper (18).

## Revendications

1. Appareil de mesure d'urine, comprenant une chambre compte-gouttes (15) dans laquelle débouche dans un goutte-à-goutte (18) et qui présente une soupape dans le fond, la soupape comprenant une ouverture de soupape (26) présentant un siège de soupape (27) périphérique au niveau de sa face inférieure et une plaque de soupape (30), et la soupape étant reliée à une chambre de mesure de précision (11), **caractérisé en ce que** la chambre compte-gouttes (15), la chambre de mesure de précision (11) et une chambre de mesure grossière (12) sont configurées dans un carter d'appareil de mesure en deux parties, qui comprend une partie de carter (20) formant une partie avant, et une partie de carter (21) formant une partie arrière, et **en ce que** l'ouverture de soupape (26) et les éléments de guidage (31) sont des éléments constitutifs de l'une des parties de carter (21) et qu'un bras en porte-à-faux (28) supportant la plaque de soupape (30) est un élément constitutif de l'autre partie de carter (20).

2. Appareil de mesure d'urine selon la revendication 1, **caractérisé en ce que** la plaque de soupape (30) est supportée par un bras en porte-à-faux (28).

3. Appareil de mesure d'urine selon la revendication 1 ou 2, **caractérisé en ce que** les éléments de guidage latéraux (31) sont des nervures saillant depuis la paroi de l'une des parties de carter (21) vers l'intérieur d'un carter de soupape (24) formé par les deux parties de carter, lequel présente une section transversale plus grande que l'ouverture de soupape (26).

4. Appareil de mesure d'urine selon l'une quelconque des revendications 1 à 3, **caractérisé en ce que** la surface de section transversale de l'ouverture de soupape (26) est plus grande d'au moins 70% que la section transversale intérieure du goutte-à-goutte (18).
